Europäisches Patentamt

European Patent Office

Office européen des brevets

(19)

(11) Numéro de publication: **0 147 339**
**A2**

(12) **DEMANDE DE BREVET EUROPEEN**

(21) Numéro de dépôt: **84420203.6**

(22) Date de dépôt: **07.12.84**

(51) Int. Cl.⁴: **A 61 F 2/46**
**A 61 B 17/16**

(30) Priorité: **08.12.83 FR 8319963**

(43) Date de publication de la demande:
**03.07.85 Bulletin 85/27**

(84) Etats contractants désignés:
**AT BE CH DE GB LI NL SE**

(71) Demandeur: **Brunet, Jean-Louis**
**Clinique Charcot 51-53 rue du Commandant Charcot**
**F-69110 Sainte-Foy-les-Lyon(FR)**

(71) Demandeur: **Brunet, André**
**14, allée de Bellevue**
**F-28130 Maintenon(FR)**

(72) Inventeur: **Brunet, Jean-Louis**
**Clinique Charcot 51-53 rue du Commandant Charcot**
**F-69110 Sainte-Foy-les-Lyon(FR)**

(72) Inventeur: **Brunet, André**
**14, allée de Bellevue**
**F-28130 Maintenon(FR)**

(54) **Dispositif pour la réalisation d'une prothèse de hanche.**

(57) Dispositif pour la réalisation d'une prothèse de hanche, du type comprenant des moyens permettant de réaliser, dans le fond de la cavité cotyloïdienne, un avant-trou (15) servant au centrage et au guidage d'un outil de restauration de la cavité, qui est monté tournant autour d'un axe passant par l'avant-trou, et une cupule dont la forme extérieure possède un profil complémentaire de celui de la cavité cotyloïdienne après restauration, caractérisé en ce qu'il comprend une tige (2) présentant une zone filetée (3) sur une partie de sa longueur, et à une extrémité de laquelle est monté un foret (4), un élément (6) en forme de douille taraudée de longueur inférieure à celle de la tige étant monté sur cette dernière, équipé, à l'une de ses extrémités, d'une fraise (8) dont le profil correspond à celui de la cavité (12) après restauration.

FIG.1

## DISPOSITIF POUR LA REALISATION D'UNE PROTHESE DE HANCHE

La présente invention a pour objet un dispositif pour la réalisation d'une prothèse de hanche.

Lors de l'implantation d'une prothèse de hanche, il convient de procéder à la mise en place d'une cupule en forme de calotte sphérique, généralement hémisphérique, dans la cavité cotyloïdienne après restauration de celle-ci, servant à l'engagement d'une tête de surface sphérique complémentaire qui, montée à l'extrémité d'une broche fixée à l'extrémité supérieure du fémur, est susceptible de pivoter par rapport à la cupule pour permettre les différents mouvements nécessaires, notamment en période de marche.

Les moyens dont disposent les chirurgiens pour réaliser la restauration de la cavité cotyloïdienne, notamment par enlèvement de matière, sont extrêmement sommaires. Il en résulte une ablation de matière osseuse souvent très supérieure à celle strictement nécessaire, la cupule étant montée dans la cavité ainsi réalisée avec interposition d'une quantité importante de ciment et de colle. Une telle solution est très critiquable. En effet, outre l'imprécision de positionnement de la cupule pouvant nuire au bon fonctionnement de la prothèse, l'utilisation massive de ciment est néfaste du fait que la température élevée de mise en oeuvre de celui-ci détériore les tissus au contact desquels il est appliqué.

La présente invention vise à remédier à ces inconvénients.

A cet effet, le dispositif qu'elle concerne comprend des moyens permettant de réaliser, dans le fond de la cavité cotyloïdienne, un avant-trou servant au centrage et au guidage d'un outil de restauration de la cavité, qui est monté tournant autour d'un axe passant par l'avant-trou, et une cupule dont la forme extérieure possède un profil complémentaire de celui de la cavité cotyloïdienne après restauration.

Ce dispositif est très intéressant car l'excellente précision de travail qu'il procure permet de réaliser l'opération de restauration avec un minimum d'ablation osseuse, et de procéder à un montage de la cupule avec un ajustement précis, ne nécessitant pas de ciment, ni de colle. Si l'on désire sceller la prothèse avec du ciment, il suffit de restaurer la cavité de façon à prévoir un léger espace entre le fond de celle-ci et la face externe de la cupule.

Selon une caractéristique de l'invention, un dispositif pour la mise en oeuvre du procédé comprend une tige présentant une zone filetée sur une partie de sa longueur, et à une extrémité de laquelle est monté un foret, un élément en forme de douille taraudée de longueur inférieure à celle de la tige étant monté sur cette dernière, équipé à l'une de ses extrémités, d'une fraise dont le profil correspond à celui de la cavité après restauration.

Dans un premier temps, la tige est positionnée pour que le foret vienne en appui dans le fond de la cavité sous l'angle de travail souhaité. L'actionnement en rotation de la tige permet alors la réalisation d'un avant-trou. Le foret demeurant dans l'avant-trou, la tige est bloquée axialement et en rotation avant réalisation d'un mouvement de rotation de la douille, qui se traduit par un mouvement d'avance et de rotation de la fraise, qui pénètre dans le cotyle avec enlèvement de matière, en vue de la restauration de la cavité.

Cette solution assure une excellente réalisation de la cavité qui est parfaitement circulaire. Il va de soi qu'il est possible de changer de fraise pour adapter la forme de la cavité à la prothèse à poser.

Selon une autre caractéristique de l'invention, la tige est montée, à proximité de son extrémité opposée à celle comportant le foret, dans un support déplaçable dans deux directions horizontales. Le réglage du support dans ces deux directions assure un parfaite positionnement angulaire de la tige et, par suite, de l'axe de travail de la fraise.

Conformément à une possibilité, la fraise, montée en bout de la douille, présente plusieurs portées cylindriques de diamètres successifs décroissants vers son extrémité libre reliés les uns aux autres par des surfaces inclinées, tandis que la cupule, destinée à être implantée dans cette cavité, présente un profil correspondant avec ménagement de nervures circulaires disposées chacune entre une partie cylindrique et une partie inclinée. Ces nervures assurent l'immobilisation de la cupule à l'intérieur de la cavité. Pour améliorer le blocage en rotation de la cupule, les nervures circulaires présentent des solutions de continuité.

Selon une autre possibilité, la fraise est conformée de façon à restaurer une cavité cotyloïdienne ne présentant pas de parties en saillie, avec ménagement d'un second trou distinct de l'avant trou ménagé selon l'axe de la cavité, ce second trou servant à l'engagement d'un doigt cylindrique, occupant la même position angulaire par rapport à la cupule que le trou par rapport à la cavité, et assurant, après engagement axial de ce doigt et pivotement de la cupule par rapport à la cavité, autour de l'axe de ce doigt, un parfait positionnement de la cupule dans la cavité. Après ce pivotement qui est de 180°, la fixation de la cupule peut être réalisée par exemple par engagement d'une vis à tête fraisée à l'intérieur d'un trou ménagé axialement dans la cupule et de l'avant-trou ménagé dans la cavité. Dans ce cas, la surface extérieure de la cupule peut être parfaitement lisse ou être striée.

Dans la mesure où la cupule est équipée, sur sa face interne, d'une coquille formant un coussinet d'usure, cette dernière peut être fixée de la même façon qu'indiqué précédemment, c'est-à-dire par coopération d'un doigt et d'un trou excentrés.

Afin d'éviter une usure prématurée de la cupule, et de favoriser le mouvement de marche, il est intéressant que l'extrémité de la tige fémorale soit équipée d'une pièce susceptible de pivoter à l'intérieur de la tête montée dans la cupule, limitant ainsi les mouvements relatifs de ces deux derniers éléments.

Selon une forme avantageuse d'exécution, la pièce solidaire de la tige fémorale est inclinée de haut en bas et de la tête de la prothèse vers la tige fémorale, selon un angle d'une valeur inférieure à 30° avec l'horizontale, lorsque le sujet est en position debout, cette pièce présentant une section non circulaire et étant elle-même engagée et bloquée en rotation dans une bague montée pivotante dans un alésage de section circulaire ménagé dans la tête de la prothèse.

Du fait de cet agencement, il se produit une conjugaison de la giration pelvienne horizontale et du mouvement pendulaire autour d'un axe correspondant à celui de la bague, concourant à la mobilité articulaire fémorale tout en réduisant les oscillations et sollicitations au niveau des interfaces de l'appui de la prothèse sur le cotyle, préservant ainsi l'ancrage cotyloïdien.

De toute façon, l'invention sera bien comprise à l'aide de la description qui suit, en référence au dessin schématique annexé représentant, à titre d'exemples non limitatifs, plusieurs formes d'exécution de dispositifs pour la mise en oeuvre de ce procédé de réalisation de prothèse :

Figure 1 est une vue en perspective, partiellement arrachée, de l'appareil destiné à la restauration cotyloïdienne, en cours d'opération ;

Figures 2 et 3 sont deux vues partiellement en perspective et partiellement en coupe de la cavité cotyloïdienne et de la cupule qu'elle est destinée à recevoir au cours de deux phases de mise en place ;

Figures 4 et 5 sont deux vues en coupe correspondant à la mise en place d'une autre cupule dans la cavité cotyloïdienne ;

Figure 6 est une vue en coupe d'une tête de prothèse ;

Figure 7 est une vue en coupe de la tête de prothèse de figure 6 équipée de l'extrémité fémorale ;

Figure 8 est une vue en coupe de cette prothèse selon la ligne 8-8 de figure 7.

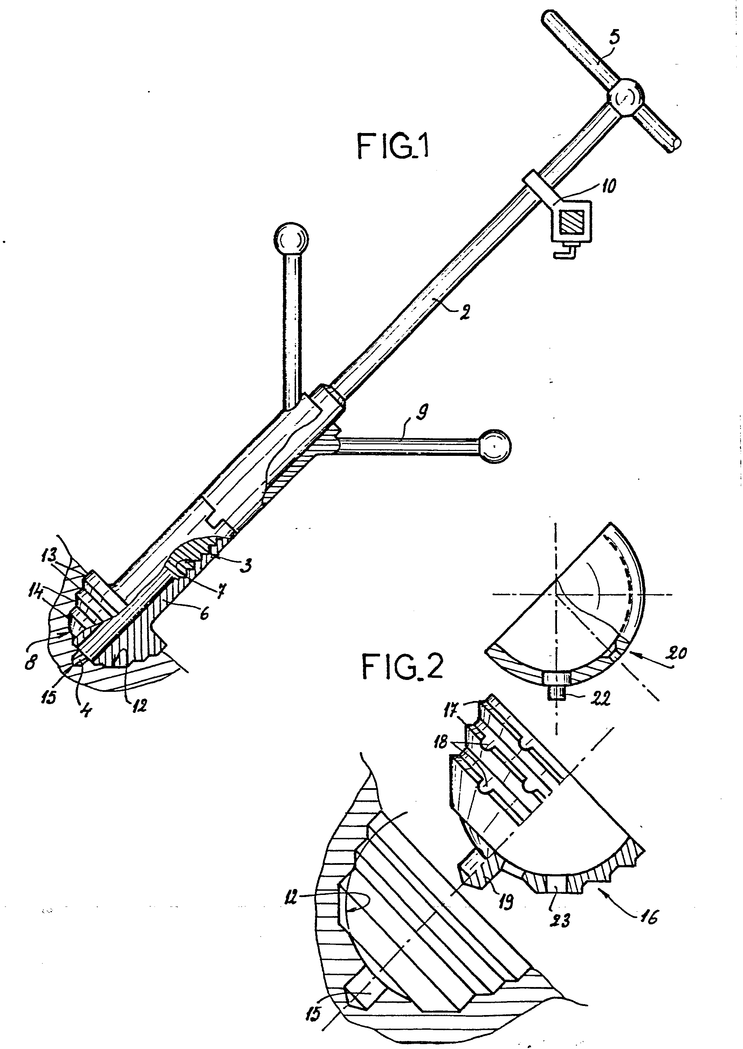

L'appareil représenté à la figure 1 comprend une tige (2) présentant un filetage (3) sur une partie de sa longueur, et équipée, à l'une de ses extrémités, d'un foret (4) et, à son autre extrémité, de poignées (5). Sur la tige (2) est engagée une douille (6), de longueur inférieure, comportant une partie taraudée (7) coopérant avec le filetage (3) de la tige, dont l'une des extrémités est en forme de fraise (8) et dont l'autre extrémité est solidaire en rotation d'une pièce d'entraînement équipée de leviers (9).

A proximité de son extrémité opposée à celle équipée d'un foret, la tige (2) repose dans une fourche (10) susceptible d'être déplacée avec possibilité de réglage dans la position désirée, dans deux directions perpendiculaires dans un plan horizontal.

Cet appareil est destiné à la restauration de la cavité cotyloïdienne (12) dont la forme générale est représentée par un trait plein de forme circulaire, en forme d'arc de cercle à la figure 2.

Dans la forme d'exécution représentée aux figures 1 à 3, la fraise comporte plusieurs portées cylindriques (13) de sections décroissantes en direction de l'extrémité libre équipée du foret (4), reliées les unes aux autres par des surfaces (14) inclinées sensiblement à 45°.

D'un point de vue pratique, il est tout d'abord procédé au positionnement du foret (4) dans le fond de la cavité cotyloïdienne (12) existante. La tige (2) est alors positionnée grâce à la fourche (10), afin que la fraise (8) travaille selon la position angulaire souhaitée. Par actionnement en rotation de la tige (2), le foret (4) réalise un trou (15) dans le fond de la cavité. Une fois cet avant-trou réalisé, la tige (2) est bloquée axialement et en rotation, et la douille (6) est entraînée en rotation par rapport à la tige, par actionnement des leviers (9), de telle sorte que la fraise (8) se déplace axialement et en rotation, afin de pénétrer dans la cavité cotyloïdienne et de restaurer celle-ci.

Une fois cette opération réalisée, la fraise est retirée, dégageant une cavité telle que représentée à la figure 2, dont il faut noter qu'elle présente un profil parfait, malgré une ablation osseuse minime. Cette cavité sert à l'engagement d'une cupule (16) dont la forme extérieure est complémentaire de celle de la cavité, avec ménagement de nervures (17) entre une surface cylindrique et la surface inclinée adjacente. Ces nervures (17) présentent des interruptions (18) favorisant un blocage en rotation de la cupule dans la cavité. La cupule présente également un doigt de centrage (19) destiné à être engagé dans l'avant-trou (15).

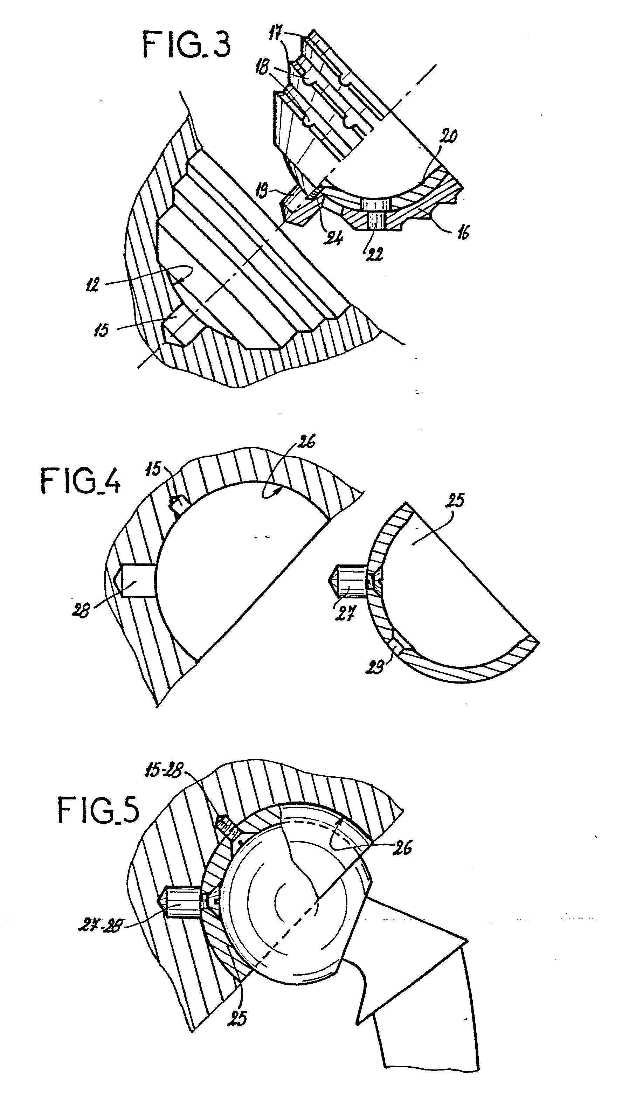

Dans la forme d'exécution représentée aux figures 2 et 3, la cupule est équipée d'une coquille (20) formant un coussinet d'usure. Cette coquille (20) présente un doigt (22) excentré faisant saillie de sa face extérieure, destiné à pénétrer dans un trou (23) qui, ménagé dans la cupule (16), occupe la même position angulaire vis-à-vis de cette dernière que le doigt (22) vis-à-vis de la coquille (20).

La coquille (20) est présentée dans la cupule (16) dans la position représentée à la figure 2, afin de réaliser un engagement axial du doigt (22) dans le trou (23). Il est ensuite procédé à un pivotement de 180° de la coquille (20) amenant celle-ci en contact parfait avec la cupule (16). La coquille peut alors être bloquée dans la cupule par une vis (24) à tête fraisée.

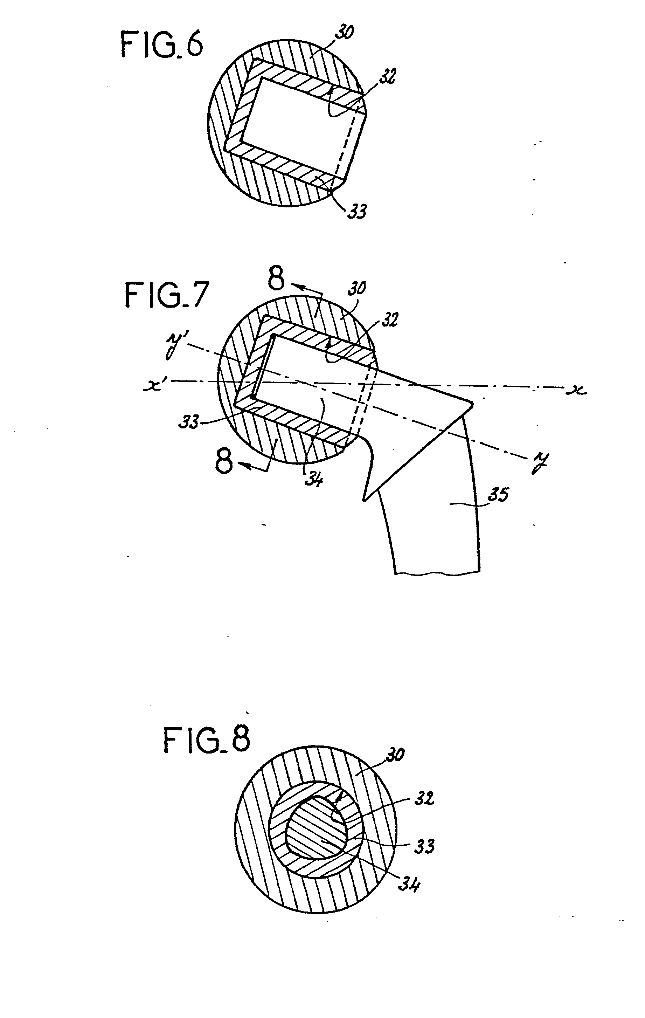

Les figures 4 et 5 représentent le montage d'une cupule (25) dans une cavité cotyloïdienne (26) restaurée de façon à présenter une paroi lisse. La cupule (25) est positionnée par engagement d'un doigt (27) pénétrant dans un trou (28) débouchant dans la cavité, par pivotement de 180° de la cupule autour du doigt (27) et blocage par une vis (28) à tête fraisée traversant un trou (29) ménagé dans la cupule et engagé dans l'avant-trou (15).

La figure 6 représente une tête (30) de forme sphérique, dans laquelle est ménagé un alésage cylindrique (32) servant au montage d'une bague (33) pivotante formant tourillon. A l'intérieur de cette bague est engagée une pièce (34) de section trigonale qui, bloquée en rotation dans la bague, est inclinée selon un axe z'z formant avec l'horizontale x'x un angle inférieur à 30°. La pièce (34) est fixée à l'extrémité supé-

rieure de la tige fémorale (35).

Comme indiqué précédemment, cet agencement, et notamment l'inclinaison de la pièce (34), permet un mouvement de marche en limitant considérablement le mouvement relatif entre la tête (30) et la cupule, respectivement (16, 25), selon les cas. La mobilité au niveau de l'axe $z'z$ soulage les cartilages de la cavité cotyloïdienne dans les applications sans cupule articulaire.

Comme il ressort de ce qui précède, l'invention apporte une grande amélioration à la technique existante en fournissant un procédé de réalisation d'une prothèse de hanche de mise en oeuvre très rigoureuse, assurant par là même une excellente fiabilité à la prothèse et un très bon confort pour le patient.

Il est intéressant de noter que ce procédé met en oeuvre des assemblages amovibles permettant le remplacement des têtes et cupules par tout type d'articulation.

Comme il va de soi, l'invention ne se limite pas au seul mode de mise en oeuvre de ce procédé, ni aux seuls dispositifs pour sa mise en oeuvre, décrits ci-dessus à titre d'exemples ; elle en embrasse, au contraire, toutes les variantes.

C'est ainsi notamment que les moyens de positionnement et de fixation de la cupule dans la cavité cotyloïdienne pourraient être différents, sans que l'on sorte pour autant du cadre de l'invention.

7

## REVENDICATIONS

1. - Dispositif pour la réalisation d'une prothèse de hanche, du type comprenant des moyens permettant de réaliser, dans le fond de la cavité cotyloïdienne, un avant-trou (15) servant au centrage et au guidage d'un outil de restauration de la cavité, qui est monté tournant autour d'un axe passant par l'avant-trou, et une cupule dont la forme extérieure possède un profil complémentaire de celui de la cavité cotyloïdienne après restauration, caractérisé en ce qu'il comprend une tige (2) présentant une zone filetée (3) sur une partie de sa longueur, et à une extrémité de laquelle est monté un foret (4), un élément (6) en forme de douille taraudée de longueur inférieure à celle de la tige étant monté sur cette dernière, équipé, à l'une de ses extrémités, d'une fraise (8) dont le profil correspond à celui de la cavité (12) après restauration.

2. - Dispositif selon la revendication 1, caractérisé en ce que la tige (2) est montée, à proximité de son extrémité opposée à celle comportant le foret (4), dans un support (10) déplaçable dans deux directions horizontales.

3. - Dispositif selon l'une quelconque des revendications 1 et 2, caractérisé en ce que la fraise (8), montée en bout de la douille (6), présente plusieurs portées cylindriques (13) de diamètres successifs décroissants vers son extrémité libre reliés les uns aux autres par des surfaces inclinées (14), tandis que la cupule (16), destinée à être implantée dans cette cavité, présente un profil correspondant avec ménagement de nervures circulaires (17) disposées chacune entre une partie cylindrique et une partie inclinée.

4. - Dispositif selon la revendication 3, caractérisée en ce que les nervures circulaires de la cupule présentent des solutions de continuité (18).

5. - Dispositif selon l'une quelconque des revendications 1 et 2, caractérisé en ce que la fraise est conformée de façon à restaurer une cavité cotyloïdienne (26) ne présentant pas de parties en saillie, avec ménagement d'un second trou (28) distinct de l'avant-trou (15) ménagé selon l'axe de la cavité, ce second trou (28) servant à l'engagement d'un doigt cylindrique (27), occupant la même position angulaire par rapport à la cupule (25) que le trou (28) par rapport à la cavité (26), et assurant, après engagement axial de ce doigt et pivotement de la cupule par rapport à la cavité, autour de l'axe de ce doigt, un parfait positionnement de

8

la cupule dans la cavité.

6. - Dispositif selon l'une quelconque des revendications 1 à 5, du type dans lequel l'extrémité de la tige fémorale est équipée d'une pièce susceptible de pivoter à l'intérieur de la tête montée dans la cupule, et inclinée de haut en bas et de la tête de la prothèse vers la tige fémorale, selon un angle d'une valeur inférieure à 30° avec l'horizontale, lorsque le sujet est en position debout, caractérisé en ce que la pièce (34) présente une section non circulaire et est elle-même engagée et bloquée en rotation dans une bague (33) montée pivotante dans un alésage (32) de section circulaire ménagé dans la tête de la prothèse.

FIG.1

FIG.2

FIG.3

FIG.4

FIG.5

FIG.6

FIG.7

FIG.8